(19) Europäisches Patentamt
European Patent Office
Office européen des brevets

(11) Veröffentlichungsnummer: **0 213 293 B1**

(12) **EUROPÄISCHE PATENTSCHRIFT**

(45) Veröffentlichungstag der Patentschrift: **02.01.92**

(51) Int. Cl.[5]: **C07D 471/04**, A61K 31/55, //(C07D471/04,243:00,221:00)

(21) Anmeldenummer: **86108098.4**

(22) Anmeldetag: **13.06.86**

Die Akte enthält technische Angaben, die nach dem Eingang der Anmeldung eingereicht wurden und die nicht in dieser Patentschrift enthalten sind.

(54) **In 11-Stellung substituierte 5,11-Dihydro-6H-pyrido-[2,3-b][1,4]benzodiazepin-6-one, Verfahren zur ihrer Herstellung und diese Verbindungen enthaltende Arzneimittel.**

(30) Priorität: **27.06.85 DE 3523002**
**03.04.86 DE 3611097**

(43) Veröffentlichungstag der Anmeldung:
**11.03.87 Patentblatt 87/11**

(45) Bekanntmachung des Hinweises auf die Patenterteilung:
**02.01.92 Patentblatt 92/01**

(84) Benannte Vertragsstaaten:
**AT BE CH DE FR GB IT LI LU NL SE**

(56) Entgegenhaltungen:
**EP-A- 0 085 893**
**EP-A- 0 125 607**
**EP-A- 0 126 366**
**EP-A- 0 156 191**

**CHEMICAL ABSTRACTS, Band 90, Nr. 17, 23. April 1979, Seite 68, abstract no. 132984s, Columbus, Ohio, US; G. HELLER et al.: "Studies on the mechanism of action of pirenzepine (gastrozepin), a new secretion inhibitor"**

(73) Patentinhaber: **Dr. Karl Thomae GmbH**
**Postfach 1755**
**W-7950 Biberach (Riss)(DE)**

(72) Erfinder: **Eberlein, Wolfgang, Dr. Dipl.-Chem.**
**Obere Au 6**
**W-7950 Biberach 1(DE)**
Erfinder: **Trummlitz, Günter, Dr. Dipl.-Chem.**
**Buchenweg 27**
**W-7951 Warthausen(DE)**
Erfinder: **Engel, Wolfhard, Dr. Dipl.-Chem.**
**Mozartstrasse 13**
**W-7950 Biberach 1(DE)**
Erfinder: **Mihm, Gerhard, Dr. Dipl.-Chem.**
**Nickeleshalde 5/1**
**W-7950 Biberach 1(DE)**
Erfinder: **Hammer, Rudolf, Dr. Biol.**
**Grundstrasse 64**
**W-6507 Ingelheim/Rhein(DE)**
Erfinder: **Mayer, Norbert, Dr. Biol.**
**Friedrich-Ebert-Strasse 66**
**W-7950 Biberach 1(DE)**
Erfinder: **Giachetti, Antonio, Prof. Dr. Pharm.**
**Via Guerrini Olindo 3**
**Mailand(IT)**

**EP 0 213 293 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **de Jonge, Adriaan, Dr. Pharm.**
**Silcherstrasse 17**
**W-7950 Biberach 1(DE)**
Erfinder: **Ballhause, Helmut**
**Fohrenweg 6**
**W-7950 Biberach 1(DE)**

## Beschreibung

Die Erfindung betrifft neue in 11-Stellung substituierte 5,11-Dihydro-6H-pyrido[2,3-b][1,4]benzodiazepin-6-one, ihre pharmakologisch verträglichen Salze mit anorganischen oder organischen Säuren, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648; 4.410.527; 4.424.225; 4.424.222 und 4.424.226 sind bereits kondensierte Diazepinone mit ulkushemmenden und magensaftsekretionshemmenden Eigenschaften bekannt.

Durch die EP 0 085 893 sind Pyrido[2,3-b][1,4]benzodiazepinone mit antiulcerogener und magensäuresekretionshemmender Wirkung, durch EP 0 125 607 und EP 0 126 366 sind Dibenzo[1,4]diazepinone, Pyrido[1,4]benzodiazepinone, Pyrido[1,5]benzodiazepinone mit antisecretorischen, antiulcerogenen, antimuscarinischen und spasmolytischen Eigenschaften bekannt.

Es wurde nun gefunden, daß die erfindungsgemäßen Diazepinone mit neuartigen Substituenten in 11-Stellung gegenüber den Verbindungen der obengenannten Publikationen überraschenderweise völlig andersartige, wertvolle pharmakologische Eigenschaften aufweisen.

Die erfindungsgemäßen Verbindungen besitzen die allgemeine Formel I

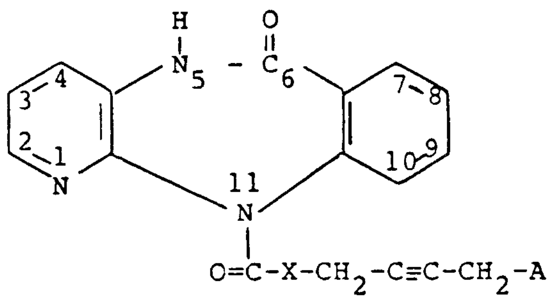

,(I)

in der
X die Gruppen

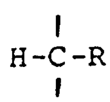
,
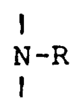

oder -0- , und
A die Gruppen

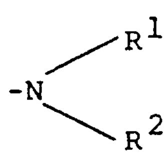
,
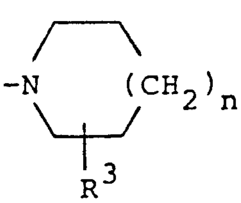
oder
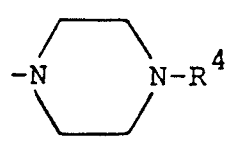

bedeuten, worin
R ein Wasserstoffatom oder eine Alkylgruppe mit 1 bis 3 Kohlenstoffatomen,
$R^1$ und $R^2$, die gleich oder voneinander verschieden sein können, ein Wasserstoffatom, einen Alkylrest mit 1 bis 3 Kohlenstoffatomen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,
n die Zahlen 0, 1 oder 2,
$R^3$ ein Wasserstoffatom, eine Hydroxygruppe, eine Alkylgruppe mit 1 bis 3 Kohlenstoffatomen oder eine Gruppe der Formel

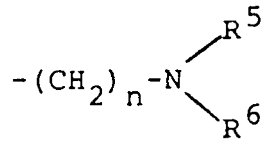,

worin n wie oben definiert ist, und $R^5$ und $R^6$ einen Alkylrest mit 1 bis 3 Kohlenstoffatomen bedeuten und $R^4$ eine gerade oder verzweigte Alkylgruppe mit 1 bis 3 Kohlenstoffatomen oder eine Phenylalkylgruppe mit insgesamt 7 bis 9 Kohlenstoffatomen darstellen.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure Zitronensäure, Maleinsäure, Bernsteinsäure, Äpfelsäure, p-Toluolsulfonsäure, Methansulfonsäure oder Amidosulfonsäure als geeignet erwiesen.

Erfindungsgemäß erhält man die Verbindungen der allgemeinen Formel I nach folgenden Verfahren:

a) durch Umsetzung einer Verbindung der allgemeinen Formel II

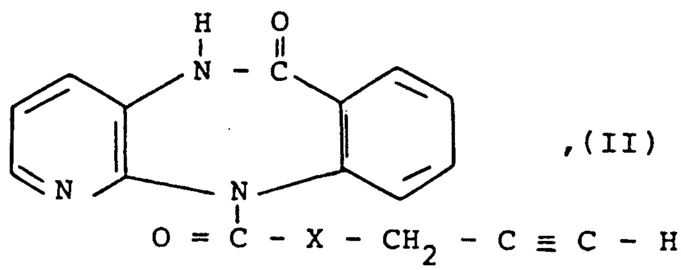

,(II)

in der X wie oben angegeben definiert ist, mit Aminen der Formeln

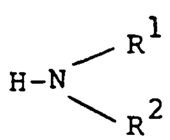,

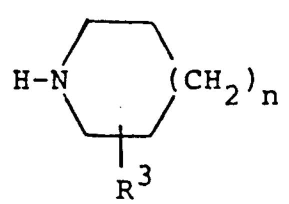,

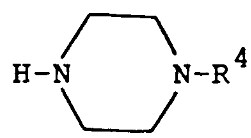

(IIIa) (IIIb) (IIIc)

worin $R^1$, $R^2$, $R^3$ und $R^4$ wie oben definiert sind,
oder mit deren Salzen in Gegenwart von Formaldehyd oder Paraformaldehyd und, gegebenenfalls, in Gegenwart katalytischer Mengen von Salzen, wie Kupfer(I)chlorid, Eisen(II)chlorid. Als Salze der Amine der Formeln IIIa, IIIb und IIIc werden vorzugsweise deren Halogenide, z.B. deren Hydrochloride, oder deren Acetate eingesetzt.

Die Umsetzung erfolgt in einem organischen Lösungsmittel bei Temperaturen bis zum Siedepunkt des Reaktionsgemisches. Als Lösungsmittel eignen sich cyclische Ether, wie Dioxan und Alkohole wie Ethylalkohol. Bei Verwendung von Dioxan empfiehlt sich der Zusatz von Essigsäure. Die Reaktion kann durch Zusatz von Salzen, wie Kupfer(I)chlorid oder Eisen(II)chlorid, beschleunigt werden. Im allgemeinen wird zuerst der Formaldehyd oder Paraformaldehyd mit einem Amin der Formeln IIIa, IIIb oder IIIc bzw. deren Salzen, z.B. deren Hydrochloriden oder Acetaten, im Lösungsmittel vereinigt und dann erst die Verbindung der allgemeinen Formel II zugegeben. Nach dem Erhitzen auf Temperaturen bis zum Rückfluß wird vom Unlöslichen abfiltriert und das Endprodukt in üblicher Weise isoliert. Im übrigen gelten die für Mannich-Reaktionen üblichen Verfahrensweisen (vgl. Weygand und Hilgetag, Organisch-Chemische Experimentierkunst, S. 990 u.f.).

b) Verbindungen der allgemeinen Formel I, in der X die Gruppe

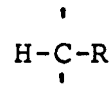

mit den für R oben angegebenen Bedeutungen ist, lassen sich auch durch Umsetzung des Dilithiumsalzes der Verbindung der Formel IV

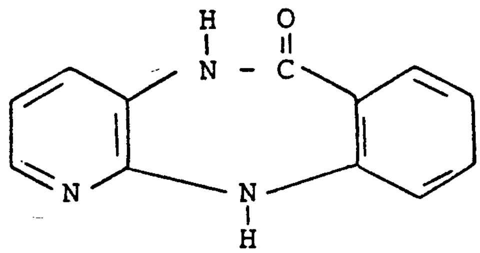

,(IV)

mit einem Ester der allgemeinen Formel V

$A-CH_2-C\equiv C-CH_2-CHR-COOR^7$ ,(V)

in der A und R wie oben definiert sind und $R^7$ einen Alkylrest mit 1 bis 10 Kohlenstoffatomen, einen Phenylalkylrest mit insgesamt 7 bis 10 Kohlenstoffatomen oder die Phenylgruppe bedeutet, herstellen.

Die Überführung des 5,11-Dihydro-6H-pyrido[2,3-b][1,4]benzodiazepin-6-ons der Formel IV in das Dilithiumsalz gelingt mit Lithiumalkylen, insbesondere aber mit n-Butyllithium, n-Butyllithium in Gegenwart von Tetramethylethylendiamin, tertiär-Butyllithium, Lithiumdiisopropylamid oder Lithiumdicyclohexylamid oder mit Lithiumarylen, z. B. mit Phenyllithium. Die Überführung in das Lithiumsalz und die weitere Umsetzung zum Endprodukt erfolgt in einem organischen Lösungsmittel bei Temperaturen zwischen -60° C und +20° C, vorzugsweise aber bei -10° C. Als organische Lösungsmittel dienen solche, die für Umsetzungen mit Lithiumreagenzien gebräuchlich sind; besonders vorteilhaft ist die Verwendung von Tetrahydrofuran oder anderen Ethern, wie Diethylether, von aliphatischen Kohlenwasserstoffen, wie Hexan oder von Gemischen hiervon, gegebenenfalls auch in Gegenwart von Hexamethylphosphorsäuretriamid als Cosolvens. Kurze Zeit nach der Beendigung der Zugabe des Metallierungs-Reagens gibt man die stöchiometrische Menge oder einen leichten Überschuß des Esters der allgemeinen Formel V hinzu und läßt das Reaktionsgemisch zur Vervollständigung der Reaktion langsam, z. B. innerhalb von 2 Stunden, auf Raumtemperatur kommen. Die gebildete Verbindung der Formel I wird nach üblichen Methoden aus dem Reaktionsgemisch isoliert und man erhält die freie Verbindung, die anschließend gewünschtenfalls in ihre Salze überführt werden kann.

Die nach diesem Verfahren erhaltenen Verbindungen der Formel I können anschließend in ihre Säureadditionssalze oder erhaltene Säureadditionssalze in die freie Basen oder andere pharmakologisch verträgliche Säureadditionssalze nach an sich bekannten Methoden übergeführt werden.

Erhält man nach den oben angeführten Verfahren Verbindungen der allgemeinen Formel I, in der X die Gruppe

$>CHR$

mit den für R oben angegebenen Bedeutungen mit Ausnahme der eines Wasserstofatoms, oder in der A eine Gruppe mit dem Rest $R_3$ darstellt, wobei $R_3$ die eingangs erwähnten Definitionen mit Ausnahme der eines Wasserstoffatoms besitzt, so können diese Verbindungen in diastereomeren Formen oder jeweils als enantiomere (+)- und (-)-Formen auftreten. Die Erfindung umfaßt die einzelnen Isomeren ebenso wie ihre Gemische. Die Trennung der jeweiligen Diastereomeren gelingt auf Grund der unterschiedlichen physikochemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Razemat einer Verbindung der allgemeinen Formel I mit einer der vorstehend angegebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit konstitutionell definierten Ausgangsverbindungen durchführt.

Die Herstellung der Ausgangsverbindungen der allgemeinen Formel II, worin X die Gruppe

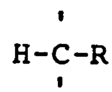

mit den oben für R genannten Bedeutungen ist, erfolgt durch Umsetzung des Dilithiumsalzes des 5,11-Dihydro-6H-pyrido[2,3-b][1,4]benzodiazepin-6-ons mit einem Pentinsäurehalogenid der allgemeinen Formel VI

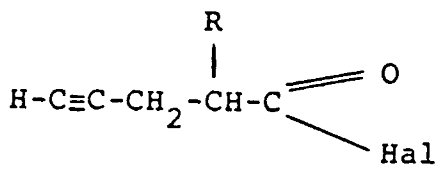
, (VI)

in der R die oben genannten Bedeutungen besitzt und Hal ein Halogenatom, vorzugsweise ein Chlor- oder Bromatom darstellt. Die Überführung des 5,11-Dihydro-6H-pyrido[2,3-b][1,4]benzodiazepin-6-ons in das Dilithiumsalz gelingt mit Lithiumalkylen, insbesondere aber mit n-Butyllithium, n-Butyllithium in Gegenwart von Tetramethylethylendiamin, tertiär-Butyllithium, Lithiumdiisopropylamid oder Lithiumdicyclohexylamid oder mit Lithiumarylen, z. B. mit Lithiumphenyl. Die Überführung in das Lithiumsalz und die weitere Umsetzung zum Endprodukt erfolgt in einem organischen Lösungsmittel bei Temperaturen zwischen -60 °C und +20 °C, vorzugsweise aber bei -10 °C. Als organische Lösungsmittel dienen solche, die für Umsetzungen mit Lithiumreagenzien gebräuchlich sind; besonders vorteilhaft ist die Verwendung von Tetrahydrofuran oder anderen Ethern, wie Diethylether, von aliphatischen Kohlenwasserstoffen, wie Hexan, oder von Gemischen hiervon, gegebenenfalls auch in Gegenwart von Hexamethylphosphorsäuretriamid als Cosolvens. Kurze Zeit nach der Beendigung der Zugabe des Metallierungs-Reagens gibt man die stöchiometrische Menge oder einen leichten Überschuß des Säurehalogenids der allgemeinen Formel VI hinzu und läßt das Reaktionsgemisch zur Vervollständigung der Reaktion langsam, z. B. innerhalb von 2 Stunden, auf Raumtemperatur kommen. Man isoliert die gebildete Verbindung der Formel II nach üblichen Methoden aus dem Reaktionsgemisch und überführt sie anschließend gewünschtenfalls in ihre Salze.

Ein Säurehalogenid der allgemeinen Formel VI läßt sich aus der entsprechenden 4-Pentinsäure durch Umsetzung mit einem Thionylhalogenid herstellen. Die Herstellung der 4-Pentinsäuren erfolt nach an sich bekannten Methoden. Die 2-Methyl-4-pentinsäure wird z. B. nach der in Bull. Soc. Chim. France, 1954, Seiten 797 und 798, beschriebenen Methode erhalten, ausgehend von Methylmalonsäurediethylester.

Die Herstellung der Ausgangsverbindungen der allgemeinen Formel II, worin X die Gruppe

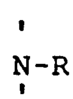

mit den oben für R genannten Bedeutungen ist, erfolgt durch Umsetzung von Pyridobenzodiazepinonen der

allgemeinen Formel VII

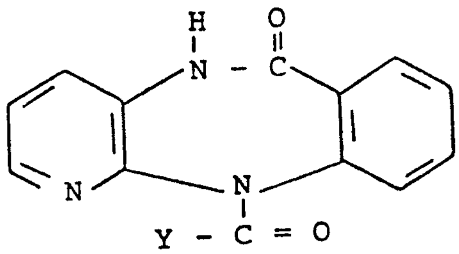

,(VII)

in der Y ein Halogenatom, bevorzugt ein Chlor- oder Bromatom, ist, mit dem Amin der allgemeinen Formel VIII

$H-C{\equiv}C-CH_2-NH-R$ ,(VIII)

worin R wie eingangs erwähnt definiert ist. Die Umsetzung wird bevorzugt in Gegenwart von Lösungsmitteln, z.B. Wasser, Toluol, Alkohole wie Methanol, Ethanol, Propanol, ganz bevorzugt aber in Gegenwart aprotischer polarer Lösungsmittel, wie Tetrahydrofuran, 1,4-Dioxan, Acetonitril, N,N-Dimethylformamid, Dimethylsulfoxid, Hexamethylphosphorsäuretriamid oder von Gemischen davon und bei Temperaturen zwischen 0 ° C und dem Siedepunkt des Reaktionsgemisches, bevorzugt zwischen 40 und 100 ° C durchgeführt. Vorteilhaft ist die Zugabe einer anorganischen oder organischen Base, wie Natriumhydroxid, Triethylamin oder Pyridin oder eines Überschusses der Base der allgemeinen Formel VIII.

Die Herstellung der Ausgangsverbindungen der allgemeinen Formel II, worin X ein Sauerstoffatom ist, erfolgt durch Umsetzung eines Pyridobenzodiazepinons der allgemeinen Formel VII mit der Verbindung der Formel IX

$H-C{\equiv}C-CH_2-OLi$ ,(IX).

Die Umsetzung wird vorzugsweise in Wasser oder Ethanol, Propanol, n-Hexan oder in aprotischen polaren Lösungsmitteln, wie Tetrahydrofuran, bei Temperaturen bis zum Siedepunkt des Reaktionsgemisches durchgeführt. Die Verbindung der Formel IX wird vorteilhafterweise in situ durch Umsetzung von Propinol mit der stöchiometrischen Menge n-Butyllithium oder Phenyllithium erzeugt, wobei sich gleich die Umsetzung mit dem Pyridobenzodiazepinon der allgemeinen Formel VII anschließen läßt.

Die Verbindungen der allgemeinen Formeln IIIa, IIIb, IIIc, und VIII sind literaturbekannt oder lassen sich in Analogie zu literaturbekannten Methoden herstellen.

Die als Zwischenprodukte erforderlichen Verbindungen der allgemeinen Formel VII erhält man durch Umsetzung des 5,11-Dihydro-6H-pyrido[2,3-b][1,4]benzodiazepin-6-ons der Formel IV mit einem Halogenkohlensäurederivat der allgemeinen Formel X

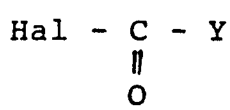

,(X)

in der Hal und Y, die nicht miteinander identisch sein müssen, ein Chlor- oder Bromatom bedeutet. Die Reaktion erfolgt in inerten Lösungsmitteln, z.B. in aromatischen Kohlenwasserstoffen, wie Toluol, Chlorbenzol, Xylol, offenkettigen oder cyclischen Ethern, wie Diisopropylether, Tetrahydrofuran oder Dioxan, in Ketonen, wie 3-Pentanon, oder in anderen Losungsmitteln, wie Acetonitril oder Dimethylformamid, bevorzugt in Gegenwart tertiärer organischer Basen, wie Pyridin, bei Temperaturen bis zum Siedepunkt des Reaktionsgemisches, bevorzugt aber zwischen 30 und 80 ° C.

Die Herstellung des 5,11-Dihydro-6H-pyrido[2,3-b][1,4]benzodiazepin-6-ons der Formel IV wird in dem US-Patent Nr. 3.406.168 beschrieben.

Ein weiterer Gegenstand der Erfindung sind Arzneimittel, die eine oder mehrere Verbindungen der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,2 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Darüber hinaus zeigt die Verbindung des Beispiels 18 an Maus und Ratte in der Versuchsanordnung nach W.W. Duke, J. Amer. Med. Ass. 15, 1185 (1910) im Dosisbereich zwischen 0,1 und 10 mg/kg nach i.v. resp. p.o. Applikation Blutungszeit-verlängernde bzw. an der Ratte in der Versuchsanordnung nach Poliwoda et al., (vgl. Seite 17) antithrombotische Eigenschaften.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

Die angewandten Methoden hatten zum Ziel, die Selektivität der antimuscarinischen Wirkung zu bestätigen.

Die Substanzen
5,11-Dihydro-11-[1-oxo-6-(1-piperidinyl)-4-hexinyl]-6H-pyrido[2,3-b][1,4]benzodiazepin-6-on = A
und
5,11-Dihydro-11-[1-oxo-6-(hexahydro-1H-azepin-1-yl)-4-hexinyl]-6H-pyrido[2,3-b][1,4]benzodiazepin-6-on = B
wurden auf Ihre

1. Tachycarde Wirkung am wachen Hund,
2. Hemmung der durch Oxotremorin stimulierten Salivation an der Ratte und
3. Mydriatische Wirkung an der Ratte

untersucht.

1. Herzfrequenz steigernde Wirkung am wachen Hund

Die Substanzen wurden intravenös injiziert und die Herzfrequenz mit Hilfe eines Tachygraphen gemessen. Nach einem Kontrollzeitraum wurden steigende Dosen der Verbindung appliziert, um die Herzfrequenz zu erhöhen. Es wurde jeweils dann die nächste Dosis injiziert, wenn der Effekt der vorangegangenen nicht mehr sichtbar war. Die Dosis einer Substanz, die eine Erhöhung um 50 Schläge/Minute ($ED_{50}$) bewirkte, wurde graphisch ermittelt. Jede Substanz wurde an 3 bis 5 Hunden untersucht.

2. Salivationshemmung an der Ratte

Verwendet wurden 10 weibliche Albino-Ratten (Stamm Crl:COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den

Vorversuchen das zu beeinflussende Symptom bei 90 bis 100 % der Tiere ausgelöst hatte.

Speichelsekretion: 0,083 mg/kg i.v.

Jede antimuscarinische Substanz wurde in gleichmäßig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d.h. der Untersucher wußte nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. 96, 99, 1949) ermittelt.

3. Mydriatische Wirkung an der Ratte

Die Mydriasis wurde durch Messung der Zunahme der Pupillenweite nach intravenöser Injektion der zu untersuchenden Substanz bestimmt. Die Messung der Pupillenweite erfolgte mit einem Mikroskop. Die Messungen wurden vor und zu verschiedenen Zeiten (15, 45 und 75 Minuten) nach der Injektion unterschiedlicher Dosen der Substanz vorgenommen. Die Resultate wurden als $ED_{200}$ ausgedrückt. Das ist jene Dosis, die eine Verdoppelung des Pupillendurchmessers, bezogen auf den Basalwert, bewirkte. Der maximale Effekt war gewöhnlich zwischen 15 und 45 Minuten nach intravenöser Gabe zu beobachten.

B Bindungsstudien an muscarinischen Rezeptoren: Bestimmung des $IC_{50}$-Wertes

Als Organspender dienten männliche Sprague-Dawley-Ratten mit 180-220 g Körpergewicht. Nach Entnahme von Herz, Magen und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 250 |
| Großhirnrinde | 1:3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 µmol $^3$H-N-Methylscopolamin ($^3$H-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 µmol Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde.

Ergebnisse:

Tabelle I

Tachycarde Wirkung am wachen Hund, Hemmung der durch Oxotremorin stimulierten Salivation an der Ratte und mydriatische Wirkung an der Ratte:

| Substanz | Tachykardie (Hund) $ED_{50}$ [µg/kg] i.v. | Salivationshemmung (Ratte) $ED_{50}$ [µg/kg] i.v. | Mydriasis (Ratte) $ED_{200}$ [µg/kg] i.v. |
|---|---|---|---|
| A | 44 | 2048 | ~ 3000 |
| B | 28 | 1136 | 2189 |

Tabelle II

Bindungsstudien an muscarinischen Rezeptoren, Bestimmung des $IC_{50}$-Wertes:

| Substanz | Rezeptor-Bindungs-Test $IC_{50}$ [nM] | |
|---|---|---|
| | Cortex | Herz |
| A | 1200 | 120 |
| B | 220 | 30 |

Aus den pharmakologischen Daten der vorstehenden Tabelle I ergibt sich, daß die Herzfrequenz durch die genannten Verbindungen bereits bei Dosierungen gesteigert wird, bei denen noch keine Einschränkung der Salivation und keine Mydriasis beobachtet werden.

Die Angaben in der vorstehenden Tabelle II beweisen, daß die neuen Verbindungen der allgemeinen Formel I zwischen muscarinischen Rezeptoren verschiedener Gewebe unterscheiden. Dies folgt aus den beträchtlich höheren $IC_{50}$-Werten bei Untersuchung an Präparaten aus der Großhirnrinde gegenüber solchen aus dem Herzen.

Die Verbindung
5,11-Dihydro-11-[1-oxo-2-methyl-6-(4-methyl-1-piperazinyl)-4-hexinyl]-6H-pyrido[2,3-b][1,4]benzodiazepin-6-on = C
die unter die allgemeine Formel I fällt und ihre Säureadditionssalze besitzen, wie bereits weiter oben angeführt, überraschenderweise auch eine stark antithrombotische Wirkung. Dies zeigt sich deutlich bei der Beeinflussung von Plättchenthromben an Ratten durch diese Substanz, die bei den Untersuchungen in Form ihres Dihydrochlorids eingesetzt wurde. Im folgenden werden diese Untersuchungen beschrieben.

Beeinflussung von Plättchenthromben an der Ratte

Es wurde im wesentlichen die von Poliwoda, Lilli, Hagemann, Schyma, in Z. ges. exp. Med. 145, 252, (1968) beschriebene Methodik angewandt.

Methodik:

Männliche SPF-Ratten (Chbb: THOM) im Gewicht von 70 bis 90 g, die bis zum Versuchsbeginn freien Zugang zu Futter (Altromin(R)) und Wasser haben, erhalten eine intraperitoneale Nembutal-Narkose (50 bis 60 mg/kg Pentobarbital-Na). Zur Vermeidung einer Hypothermie werden sie auf einem geheizten Versuchstisch (37° C) gelagert.

Nach Anlegen eines transversalen Bauchschnittes wird eine Dünndarmschlinge vorgelagert, fixiert und eine Mesenterialvene mit einem Durchmesser von 300µm unter einem binokularen Operationsmikroskop mit 40facher Vergrößerung eingestellt. An die Vene wird eine in Glas eingebettete monopolare V4A-Stahl-Elektrode von 100 µm Spitzendurchmesser als Reizkathode angelegt. Die Anode liegt am gleichen Gefäß der Kathode gegenüber. Die Reizung geschieht mit 150 Volt Gleichstrom für die Dauer von 100 µsec. Nach der Reizung wird das Beobachtungsgebiet kontinuierlich mit warmer (37° C) physiologischer Kochsalzlösung bespült.

Entstehung und Wachstum des Thrombus wird unter dem Mikroskop über einen Zeitraum von 20 Minuten verfolgt. Während der gesamten Beobachtungsdauer wird anfangs im 10 sec-Abstand, später jede Minute die prozentuale Einengung des Gefäßdurchmessers durch den Thrombus ermittelt und protokolliert.

Die Werte werden gegen die Zeit in ein Koordinatensystem eingetragen. Die Fläche unter der so erhaltenen Kurve ("area under curve") stellt ein Maß für die Thrombusgröße über die Zeit dar.

Eine Gruppe von 5 Tieren erhält 5 Minuten vor Beobachtungsbeginn 1 mg/kg der Substanz C als Dihydrochlorid intravenös verabreicht (0,1 ml pro 100 g Körpergewicht, Lösungsmittel Aqua dest.). Eine Kontrollgruppe von ebenfalls 5 Tieren erhält bei sonst gleicher Behandlung das Vehikel intravenös.

Die "area under curve" der substanzbehandelten Tiere wird mit der der Kontrolltiere verglichen. Die Reduktion der Thrombusgröße unter Substanzeinfluß wird erfaßt als prozentuale Verminderung des Mittelwertes der "area under curve" der substanzbehandelten Tiere über die gesamte Beobachtungszeit gegenüber dem Mittelwert der Kontrolle.

Die Signifikanz der Verminderung wird berechnet nach dem t-Test für unabhängige Stichproben nach STUDENT (CAVALLI-SFORZA: Biometrie, G. Fischer-Verlag, Stuttgart 1969, Seite 72).

Ergebnis:

Substanz C

| Behandlung | Dosis mg/kg i.v. | n | "area under curve" Flächeneinheiten MW ± SD | Prozentuale Verminderung gegen Kontrollen |
|---|---|---|---|---|
| Kontrolle | - | 5 | 1736,0 ± 124,2 | |
| Substanz C als Dihydrochlorid | 1 | 5 | 1230,0 ± 188,0$^x$ | 29,2 |

$^x$ signifikante Verminderung, t-Test, $p < 0,02$.

Das Dihydrochlorid der Substanz C vermindert in der Dosierung 1 mg/kg i.v. die Größe der elektrisch induzierten Mesenterialthromben bei Ratten hochsignifikant um 29,2%. Es wurde also überraschenderweise gefunden, daß diese Substanz eine ausgeprägte antithrombotische Wirkung entfaltet.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die nachfolgenden Beispiele sollen die Erfindung näher er-läutern:

Herstellung der Ausgangsmaterialien

Beispiel A

5,11-Dihydro-11-[1-oxo-4-pentinyl]-6H-pyrido[2,3-b][1,4]benzodiazepin-6-on

Zu einer Suspension von 3,7 g (0,017 Mol) 5,11-Dihydro-6H-pyrido[2,3-b][1,4]benzodiazepin-6-on in 150 ml absolutem Tetrahydrofuran werden bei 0 °C unter Rühren 22,4 ml einer 1,6 molaren Lösung von n-Butyllithium in n-Hexan getropft. Nach beendeter Zugabe wird das Gemisch 30 Minuten weiter gerührt und anschließend mit einer Lösung von 2,04 g (0,0175 Mol) 4-Pentinsäurechlorid in 20 ml Tetrahydrofuran versetzt. Man erwärmt den Ansatz auf 30 °C und rührt eine Stunde weiter. Danach wird das Reaktionsgemisch in eine gesättigte Kochsalz-Lösung eingetragen. Die organische Phase wird mit Essigester verdünnt und nach Abtrennung zweimal mit Kochsalzlösung gewaschen, über Kohle filtriert und im Vakuum zur Trockne eingeengt. Das Rohprodukt wird säulenchromatographisch an Kieselgel unter Verwendung von Chloroform als Elutionsmittel gereinigt. Man erhält 2.2 g (43 % der Theorie) farblose Kristalle vom Schmelzpunkt 179-180 °C.

Beispiel B

5,11-Dihydro-11-[2-methyl-1-oxo-4-pentinyl]-6H-pyrido[2,3-b][1,4]benzodiazepin-6-on

Analog Beispiel A werden unter Verwendung von 63 g (0,3 Mol) 5,11-Dihydro-6H-pyrido[2,3-b][1,4]-benzodiazepin-6-on und 46,8 g (0,36 Mol) 4-(2-Methyl)-pentinsäurechlorid 88 g (96% der Theorie) der gewünschten Verbindung vom Schmelzpunkt 202-204 °C erhalten.

Beispiel C

5,11-Dihydro-11-[[2-propinyl)amino]carbonyl]-6H-pyrido[2,3-b][1,4]benzodiazepin-6-on

Zu einer Suspension von 27,3 g (0,1 Mol) 11-Chlorcarbonyl-5,11-dihydro-6H-pyrido[2,3-b][1,4]-benzodiazepin-6-on und 10,0 g (0,1 Mol) Triäthylamin in 500 ml Chloroform werden unter Rühren bei Raumtemperatur 5,5 g (0,1 Mol) Propargylamin getropft, wobei die Temperatur auf 35 °C ansteigt. Man rührt bei 40 °C 30 Minuten weiter, filtriert anschließend über Aktivkohle und engt die Reaktionslösung im Vakuum zur Trockne ein. Nach dem Aufkochen des öligen Rückstandes mit Essigester erhält man das gewünschte Produkt als farblose Kristallmasse in einer Ausbeute von 20,7 g (71% der Theorie).

Beispiel D

5,11-Dihydro-11-[[N-methyl(2-propinyl)amino]carbonyl]-6H-pyrido[2,3-b][1,4]benzodiazepin-6-on

Analog Beispiel C werden unter Verwendung von 27,3 g (0,1 Mol) 11-Chlorcarbonyl-5,11-dihydro-6H-pyrido[2,3-b][1,4]benzodiazepin-6-on und 7,0 g (0,1 Mol) N-Methyl-propargylamin 27 g (88% der Theorie) der gewünschten Verbindung erhalten.

Beispiel E

5,11-Dihydro-11-[[(2-propinyl)oxy]carbonyl]-6H-pyrido[2,3-b][1,4]benzodiazepin-6-on

Zu einer Lösung von 2,24 g (0,04 Mol Propargylalkohol in 120 ml Tetrahydrofuran werden bei 15-20 °C 3,08 g (0,048 Mol) n-Butyllithium in 30 ml n-Hexan getropft. Nach beendeter Zugabe wird das Gemisch bei Raumtemperatur 30 Minuten weiter gerührt und anschließend mit einer Suspension von 10,9 g (0,04 Mol) 11-Chlorcarbonyl-5,11-dihydro-6H-pyrido[2,3-b][1,4]benzodiazepin-6-on in 250 ml Tetrahydrofuran versetzt. Zur Vervollständigung der Reaktion wird der Ansatz eine Stunde weiter gerührt. Man engt im Vakuum zur Trockne ein und bringt den Rückstand durch Einrühren in Wasser zur Kristallisation. Nach dem Umkristallisieren aus Essigester erhält man 7,5 g (64 % der Theorie) des gewünschten Produktes vom Schmelzpunkt 213 °C.

Beispiel F

5,11-Dihydro-11-[1-oxo-2-methyl-4-pentinyl]-6H-pyrido[2,3-b][1,4]benzodiazepin-6-on

a.) 2-Methyl-2-propargyl-malonsäurediethylester

In eine Alkoholatlösung (hergestellt aus 23 g Natrium und 800 ml absolutem Ethanol) tropft man bei 35-40° C unter Rühren 174 g (1,0 Mol) Methylmalonsäurediethylester ein, rührt noch eine Stunde nach und läßt anschließend bei 45° C 130,8 g (1,1 Mol) Propargylbromid zutropfen. Der Ansatz wird weitere zwei Stunden unter Rückfluß gerührt. Nach dem Erkalten wird vom ausgefallenen Natriumbromid abgesaugt und das Filtrat im Vakuum weitgehend eingeengt. Der Rückstand wird in Eiswasser eingerührt und die wässerige Lösung mehrmals mit Ether extrahiert. Die vereinigten organischen Phasen werden getrocknet und im Vakuum zur Trockne eingedampft. Das resultierende Öl setzt man ohne weitere Reinigung in der nächsten Stufe ein.
Ausbeute: 187 g (88% der Theorie).

b.) 2-Methyl-2-propargyl-malonsäure

Zu einer Lösung von 101 g (1,8 Mol) Kaliumhydroxid in einer Mischung von 560 ml Wasser/Ethanol (1:1) gibt man 187 g (0,88 Mol) 2-Methyl-2-propargyl-malonsäurediethylester und erhitzt den Ansatz unter Rühren während zwei Stunden zum Rückfluß. Nach beendeter Reaktion (DC-Kontrolle) engt man im Vakuum zur Trockne ein und nimmt anschließend den Rückstand in Wasser auf. Die wässerige Lösung wird mit 50%iger Schwefelsäure angesäuert und mehrmals mit Methylenchlorid extrahiert. Man trocknet die vereinigten Auszüge, engt im Vakuum zur Trockne ein und erhält ein farbloses Produkt vom Schmelzpunkt 135° C.
Ausbeute: 135 g (98% der Theorie).

c.) 2-Methyl-4-pentinsäure

62,4 g (0,4 Mol) 2-Methyl-2-propargyl-malonsäure werden imÖlbad bei 180° C bis zur Beendigung der $CO_2$-Entwicklung erhitzt (ca. 30 Minuten). Man erhält ein gelbliches Öl welches direkt in der nächsten Stufe weiterverarbeitet wird.
Ausbeute: 41 g (91,5% der Theorie).

d.) 2-Methyl-4-pentinsäurechlorid

40 g (0,36 Mol) 2-Methyl-4-pentinsäure werden in 85 g Thionylchlorid gelöst und während 24 Stunden bei Raumtemperatur gerührt. Man destilliert anschließend vom Thionylchlorid ab und setzt das erhaltene Säurechlorid ohne weitere Reinigung direkt in der nächsten Stufe ein.
Ausbeute: 46,8 g ( 100% der Theorie).

e.) 5,11-Dihydro-11-[1-oxo-2-methyl-4-pentinyl]-6H-pyrido[2,3-b][1,4]benzodiazepin-6-on

Zu einer Suspension von 63 g (0,3 Mol) 5,11-Dihydro-6H-pyrido[2,3-b][1,4]benzodiazepin-6-on in 1500 ml absolutem Tetrahydrofuran werden unter Rühren bei 0-10° C 416 ml (0,65 Mol) einer n-Buthyllithiumlösung in n-Hexan zugetropft. Man rührt bei Raumtemperatur eine Stunde weiter, kühlt dann auf 5-10° C ab und tropft bei dieser Temperatur 46,8 g (0,36 Mol) 2-Methyl-4-pentinsäurechlorid (nicht gereinigt), gelöst in 100 ml absolutem Tetrahydrofuran, in die Reaktionslösung ein. Der Ansatz wird noch drei Stunden bei Raumtemperatur weitergerührt, anschließend in 2000 ml einer gesättigten Kochsalzlösung eingetragen und mit ca. 2000 ml Essigester verdünnt. Man trennt die organische Phase ab, extrahiert sie zweimal mit jeweils 500 ml gesättigter Kochsalzlösung, trocknet über Magnesiumsulfat und engt im Vakuum zur Trockene ein. Man erhält die gewünschte Verbindung als gelbbraunes Produkt, das ohne weitere Reinigung in der nächsten Stufe weiter verwendet wird.
Rohausbeute: 88 g (96% der Theorie).

Eine kleine Probe wurde säulenchromatographisch gereinigt.
Fp.: 202-204° C (Ether).

Herstellung der Endprodukte

Beispiel 1

5,11-Dihydro-11-[1-oxo-6-(1-piperidinyl)-4-hexinyl]-6H-pyrido[2,3-b][1,4]benzodiazepin-6-on

Eine Mischung bestehend aus 9,6 g (0,03 Mol) 5,11-Dihydro-11-[1-oxo-4-pentinyl]-6H-pyrido[2,3-b][1,4]-benzodiazepin-6-on, 1,08 g (0,036 Mol) Paraformaldehyd, 3,06 g (0,036 Mol) Piperidin, 0,2 g Kupfer(I)chlorid und 150 ml Dioxan wird eine Stunde unter Rückfluß erhitzt. Nach beendeter Reaktion filtriert man von unlöslichen Bestandteilen ab und engt das Filtrat im Vakuum zur Trockne ein. Das Rohprodukt wird säulenchromatographisch an Kieselgel (Mobile Phase: Essigester, Essigester +10% Methanol) gereinigt. Nach dem Umkristallisieren aus Essigester erhält man 2,7 g (21% der Theorie) der gewünschten Verbindung vom Schmelzpunkt 197° C.

Beispiel 2

5,11-Dihydro-11-[1-oxo-6-(1-pyrrolidinyl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und Pyrrolidin in einer Ausbeute von 41% der Theorie;
Schmelzpunkt: 185-186° C (Essigester).

Beispiel 3

5,11-Dihydro-11-[1-oxo-6-(hexahydro-1H-azepin-1-yl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und Hexamethylenimin in einer Ausbeute von 24% der Theorie;
Schmelzpunkt: 169-170° C (Essigester).

Beispiel 4

5,11-Dihydro-11-[1-oxo-6-(4-methyl-1-piperazinyl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 4-Methyl-piperazin in einer Ausbeute von 25% der Theorie;
Schmelzpunkt: 182-183° C.

Beispiel 5.

5,11-Dihydro-11-[1-oxo-6-[(methyl)(cyclohexyl)amino]-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und N-Cyclohexyl-methylamin in einer Ausbeute von 20% der Theorie;
Schmelzpunkt: 162-163° C (Essigester).

Beispiel 6

5,11-Dihydro-11-[1-oxo-6-(diethylamino)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und Diethylamin in einer Ausbeute von 16% der Theorie;
Schmelzpunkt: 173-174° C (Essigester).

Beispiel 7

5,11-Dihydro-11-[1-oxo-6-(4-trans-hydroxy-1-piperidinyl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 4-Hydroxy-piperidin in einer Ausbeute von 30% der Theorie;
Schmelzpunkt: 175-176° C.

Beispiel 8

5,11-Dihydro-11-[1-oxo-6-[[2-(diethylamino)methyl]-1-piperidinyl]-4-hexinyl]-6H-pyrido[2,3-b][1,4]-benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 2-[(Diethylamino)methyl]piperidin in einer Ausbeute von 14% der Theorie;
Schmelzpunkt: 157-158° C (Essigester).

Beispiel 9

5,11-Dihydro-11-[1-oxo-6-[(methyl)(4-trans-hydroxy-cyclohexyl)amino]-4-hexinyl]-6H-pyrido[2,3-b][1,4]-benzodiazepin-6-on

Hergestellt analog Beispiel 1 aus 5,11-Dihydro-11-[1-oxo-4-pentinyl]-6H-pyrido[2,3-b][1,4]benzodiazepin-6-on und N-(4-trans-hydroxy)-cyclohexyl-methylamin in einer Ausbeute von 27% der Theorie;
Schmelzpunkt: 176-178° C (Essigester).

Beispiel 10

5,11-Dihydro-11-[1-oxo-6-(4-trans-hydroxy-hexahydro-1H-azepin-1-yl)-4-hexinyl]-6H-pyrido[2,3-b][1,4]-benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 4-Hydroxy-hexamethylenimin in einer Ausbeute von 16% der Theorie;
Schmelzpunkt: 140-141° C.

Beispiel 11

5,11-Dihydro-11-[1-oxo-6-[(methyl)(benzyl)amino]-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und N-Benzyl-methylamin in einer Ausbeute von 11% der Theorie;
Schmelzpunkt 204-206° C (Essigester).

Beispiel 12

5,11-Dihydro-11-[1-oxo-6-(4-isopropyl-1-piperazinyl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 4-Isopropyl-piperazin in einer Ausbeute von 10% der Theorie;
Schmelzpunkt: 124-126° C (Diisopropylether/Essigester).

Beispiel 13

5,11-Dihydro-11-[1-oxo-6-(4-benzyl-1-piperazinyl)-4-hexinyl]-6H-pyrido[2,3-b][1,4]benzodiazepin-6-on

Hergestellt analog Beispiel 1 aus 5,11-Dihydro-11-[1-oxo-4-pentinyl]-6H-pyrido[2,3-b][1,4]benzodiazepin-6-on und 4-Benzyl-piperazin in einer Ausbeute von 21% der Theorie;
Schmelzpunkt: 157-158° C (Essigester).

Beispiel 14

5,11-Dihydro-11-[1-oxo-2-methyl-6-(1-pyrrolidinyl)-4-hexinyl]-6H-pyrido[2,3-b][1,4]benzodiazepin-6-on

Hergestellt analog Beispiel 1 aus 5,11-Dihydro-11-[2-methyl-1-oxo-4-pentinyl]-6H-pyrido[2,3-b][1,4]-benzodiazepin-6-on und Pyrrolidin in einer Ausbeute von 52% der Theorie;
Schmelzpunkt: 158° C (Essigester).

Beispiel 15

5,11-Dihydro-11-[[[4-(1-piperidinyl)-2-butinyl]amino]carbonyl]-6H-pyrido[2,3-b][1,4]benzodiazepin-6-on

Hergestellt analog Beispiel 1 aus 5,11-Dihydro-11-[[(2-propinyl)amino]carbonyl]-6H-pyrido[2,3-b][1,4]-benzodiazepin-6-on und Piperidin in einer Ausbeute von 44% der Theorie;
Schmelzpunkt: 199-200 °C (Essigester/Ethanol).

Beispiel 16

5,11-Dihydro-11-[[[4-(hexahydro-1H-azepin-1-yl)-2-butinyl](methyl)amino]carbonyl]-6H-pyrido[2,3-b][1,4]-benzodiazepin-6-on

Hergestellt analog Beispiel 1 aus 5,11-Dihydro-11-[[N-methyl(2-propinyl)amino]carbonyl]-6H-pyrido[2,3-b][1,4]benzodiazepin-6-on und Hexamethylenimin in einer Ausbeute von 26% der Theorie;
Schmelzpunkt: 145-146 °C (Ether).

Beispiel 17

5,11-Dihydro-11-[[[4-(1-piperidinyl)-2-butinyl]oxy]carbonyl]-6H-pyrido[2,3-b][1,4]benzodiazepin-6-on

Hergestellt analog Beispiel 1 aus 5,11-Dihydro-11-[[2-propinyl)oxy]carbonyl]-6H-pyrido[2,3-b][1,4]-benzodiazepin-6-on und Piperidin in einer Ausbeute von 22% der Theorie;
Schmelzpunkt: 207-208 °C (Zersetzung).

Beispiel 18

5,11-Dihydro-11-[1-oxo-2-methyl-6-(4-methyl-1-piperazinyl)-4-hexinyl]-6H-pyrido[2,3-b][1,4]benzodiazepin-6-on

Eine Reaktionsmischung, bestehend aus 36,6 g (0,12 Mol) 5,11-Dihydro-11-[1-oxo-2-methyl-4-pentinyl]-6H-pyrido[2,3-b][1,4]benzodiazepin-6-on, 4 g (0,13 Mol) Paraformaldehyd, 13,2 g (0,13 Mol) N-Methylpiperazin, 0,2 g Kupfer(I)chlorid und 600 ml Dioxan, wird zwei Stunden unter Rückfluß erhitzt. Nach beendeter Reaktion filtriert man über Aktivkohle von unlöslichen Bestandteilen ab und engt das Filtrat im Vakuum zur Trockne ein. Zur Reinigung wird das Rohprodukt in einer Lösung von 15,4 g (0,13 Mol) Maleinsäure in 600 ml Wasser aufgeschlämmt und eine Stunde bei Raumtemperatur gerührt. Man filtriert von unlöslichen Bestandteilen ab, stellt das Filtrat durch Zugabe von Kaliumkarbonat alkalisch und nimmt das ausgefallene Harz in 600 ml Methylenchlorid auf. Nach dem Trocknen über Magnesiumsulfat engt man die Lösung im Vakuum zur Trockne ein. Der kristalline Rückstand wird mit 150 ml Essigester aufgekocht und nach dem Abkühlen abgesaugt. Durch Digerieren in wenig Essigester erhält man ein kristallines Produkt, das nach dem Waschen mit Ether bei 212-214 °C schmilzt.
Ausbeute: 30,5 g (60,9% der Theorie).

Beispiel 19

5,11-Dihydro-11-[1-oxo-2-methyl-6-(4-methyl-1-piperazinyl)-4-hexinyl]-6H-pyrido[2,3-b][1,4]benzodiazepin-6-on-dihydrochlorid

28 g (0,067 Mol) 5,11-Dihydro-11-[1-oxo-2-methyl-6-(4-methyl-1-piperazinyl)-4-hexinyl]-6H-pyrido[2,3-b]-[1,4]benzodiazepin-6-on werden in 350 ml absolutem Ethanol aufgeschlämmt und unter Rühren in der Siedehitze mit 45 ml etherischer Salzsäure versetzt. Man erhält vorübergehend eine klare Lösung, die sich nach einiger Zeit wieder eintrübt. Es kommt zur Abscheidung eines feinen Kristallbreis, den man nach dem Abkühlen absaugt und mit 50 ml absolutem Ethanol und 150 ml Aceton nachwäscht.
Fp.: 220-221 °C.
Ausbeute: 32,5 g (98,9% der Theorie).

Im folgenden wird die Herstellung pharmazeutischer Anwendungsformen anhand einiger Beispiele beschrieben:

Beispiel I

Tabletten mit 5 mg 5,11-Dihydro-11-[1-oxo-6-(1-piperidinyl)-4-hexinyl]-6H-pyrido[2,3-b][1,4]benzodiazepin-6-on

Zusammensetzung:
1 Tablette enthält:

| | |
|---|---|
| Wirkstoff | 50,0 mg |
| Milchzucker | 148,0 mg |
| Kartoffelstärke | 65,0 mg |
| Magnesiumstearat | 2,0 mg |
| | 265,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg 5,11-Dihydro-11-[1-oxo-6-(1-piperidinyl)-4-hexinyl]-6H-pyrido[2,3-b][1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 1 mg 5,11-Dihydro-11-[1-oxo-6-(1-piperidinyl)-4-hexinyl]-6H-pyrido[2,3-b][1,4]benzodiazepin-6-on

Zusammensetzung:
1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120 °C.

Beispiel IV

Suppositorien mit 5 mg 5,11-Dihydro-11-[1-oxo-6-(1-piperidinyl)-4-hexinyl]-6H-pyrido[2,3-b][1,4]-benzodiazepin-6-on

Zusammensetzung:
1 Zäpfchen enthält:

| | |
|---|---|
| Wirkstoff | 50,0 mg |
| Zäpfchenmasse (z.B. Witepsol W 45$^{(R)}$) | 1 695,0 mg |
| | 1 745,0 mg |

Herstellungsverfahren:

Die feinpulverisierte Wirksubstanz wird in der geschmolzener und auf 40 °C abgekühlten Zäpfchenmasse suspendiert. Man gießt die Masse bei 37 °C in leicht vorgekühlte Zäpfchenformen aus.
Zäpfchengewicht 1,7 g

Beispiel V

Tropfen mit 5,11-Dihydro-11-[1-oxo-6-(1-piperidinyl)-4-hexinyl]-6H-pyrido[2,3-b][1,4]benzodiazepin-6-on

Zusammensetzung:
100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 5,0 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesaureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Beispiel VI

Tabletten mit 50 mg 5,11-Dihydro-11-[1-oxo-2-methyl-6-(4-methyl-1-piperazinyl)-4-hexinyl]-6H-pyrido[2,3-b]-[1,4]-benzodiazepin-6-on

Zusammensetzung:
1 Tablette enthält:

| | |
|---|---|
| Wirkstoff | 50,0 mg |
| Milchzucker | 148,0 mg |
| Kartoffelstärke | 65,0 mg |
| Magnesiumstearat | 2,0 mg |
| | 265,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel VII

Dragées mit 50 mg 5,11-Dihydro-11-[1-oxo-2-methyl-6-(4-methyl-1-piperazinyl)-4-hexinyl]-6H-pyrido[2,3-b]-[1,4]-benzodiazepin-6-on

Die nach Beispiel VI hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel VIII

Ampullen mit 10 mg 5,11-Dihydro-11-[1-oxo-2-methyl-6-(4-methyl-1-piperazinyl)-4-hexinyl]-6H-pyrido[2,3-b]-[1,4]-benzodiazepin-6-on-dihydrochlorid

Zusammensetzung:
1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das

gegebene Volumen aufgefüllt. Die Lösung wird sterilfiltriert und in 1 ml-Ampullen abgefüllt.
Sterilisation: 20 Minuten bei 120 °C.

Beispiel IX

Suppositorien mit 50 mg 5,11-Dihydro-11-[1-oxo-2-methyl-6-(4-methyl-1-piperazinyl)-4-hexinyl]-6H-pyrido-[2,3-b][1,4]-benzodiazepin-6-on

Zusammensetzung:

1 Zäpfchen enthält:

| | |
|---|---|
| Wirkstoff | 50,0 mg |
| Zäpfchenmasse (z.B. Witepsol W 45$^{(R)}$) | 1 695,0 mg |
| | 1 745,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht 1,745 g

Beispiel X

Tropfen mit 5,11-Dihydro-11-[1-oxo-2-methyl-6-(4-methyl-1-piperazinyl)-4-hexinyl]-6H-pyrido[2,3-b][1,4]-benzodiazepin-6-on-dihydrochlorid

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 5,0 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche**

**Patentansprüche für folgenden Vertragsstaaten: BE, CH, DE, FR, GB, IT, LI, LU, NL, SE**

**1.** In 11-Stellung substituierte 5,11-Dihydro-6H-pyrido[2,3-b][1,4]benzodiazepin-6-one der allgemeinen Formel I

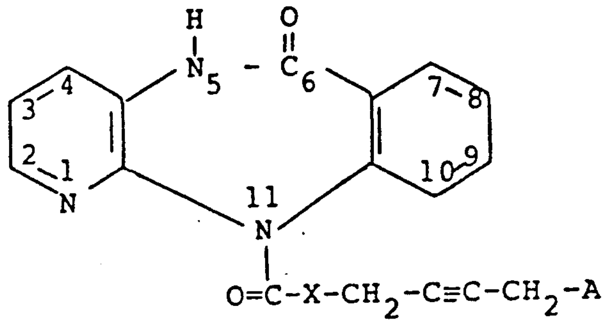 ,(I)

in der

X die Gruppen

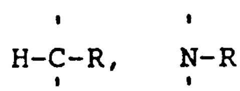

oder -0- , und

A die Gruppen

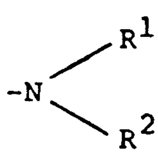 , 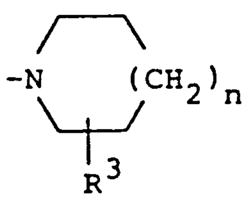 oder 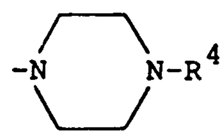

bedeuten, worin

R ein Wasserstoffatom oder eine Alkylgruppe mit 1 bis 3 Kohlenstoffatomen,

$R^1$ und $R^2$, die gleich oder voneinander verschieden sein können, ein Wasserstoffatom, einen Alkylrest mit 1 bis 3 Kohlenstoffatonen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,

n die Zahlen 0, 1 oder 2,

$R^3$ ein Wasserstoffatom, eine Hydroxygruppe, eine Alkylgruppe mit 1 bis 3 Kohlenstoffatomen oder eine Gruppe der

Formel 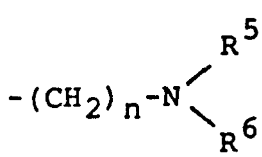 ,

worin n wie oben definiert ist, und $R^5$ und $R^6$ einen Alkylrest mit 1 bis 3 Kohlenstoffatomen bedeuten und

$R^4$ eine gerade oder verzweigte Alkylgruppe mit 1 bis 3 Kohlenstoffatomen oder eine Phenylalkylgruppe mit insgesamt 7 bis 9 Kohlenstoffatomen darstellen, ihre Isomere und ihre pharmakologisch verträglichen Salze mit anorganischen oder organischen Säuren.

**2.** 5,11-Dihydro-11-[1-oxo-2-methyl-6-(4-methyl-1-piperazinyl)-4-hexinyl]-6H-pyrido[2,3-b][1,4]-benzodiazepin-6-on und dessen pharmakologisch verträgliche Säureadditionssalze mit anorganischen oder organischen Säuren.

**3.** 5,11-Dihydro-11-[1-oxo-6-(1-piperidinyl)-4-hexinyl]-6H-pyrido[2,3-b][1,4]benzodiazepin-6-on und seine pharmakologisch verträglichen Salze mit anorganischen oder organischen Säuren.

**4.** 5,11-Dihydro-11-[1-oxo-6-(hexahydro-1H-azepin-1-yl)-4-hexinyl]-6H-pyrido[2,3-b][1,4]benzodiazepin-6-on und seine pharmakologisch verträglichen Salze mit anorganischen oder organischen Säuren.

**5.** Arzneimittel enthaltend eine Verbindung der allgemeinen Formel I gemäß Anspruch 1 neben üblichen Träger- und/oder Hilfsstoffen.

**6.** Arzneimittel enthaltend 5,11-Dihydro-11-[1-oxo-2-methyl-6-(4-methyl-1-piperazinyl)-4-hexinyl]-6H-pyrido[2,3-b][1,4]benzodiazepin-6-on und/oder dessen pharmakologisch verträgliche Säureadditionssalze mit anorganischen oder organischen Säuren neben üblichen Hilfs- und Zusatzstoffen.

**7.** Verwendung von 5,11-Dihydro-11-[1-oxo-2-methyl-6-(4-methyl-1-piperazinyl)-4-hexinyl]-6H-pyrido[2,3-b][1,4]benzodiazepin-6-on und/oder dessen pharmakologisch verträglichen Säureadditionssalzen mit anorganischen oder organischen Säuren zur Herstellung eines Arzneimittels mit antithrombotischen Eigenschaften zur Behandlung von thrombo-embolischen Erkrankungen.

**8.** Verwendung von Verbindungen der Ansprüche 1, 3 und 4 zur Herstellung eines Arzneimittels zur Behandlung von Bradycardien und Bradyarrhythmien.

**9.** Verfahren zur Herstellung von in 11-Stellung substituierten 5,11-Dihydro-6H-pyrido[2,3-b][1,4]-benzodiazepin-6-onen der allgemeinen Formel I

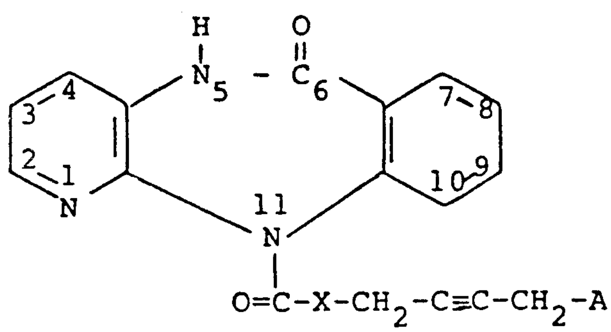

,(I)

in der

X die Gruppen

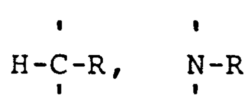

oder -0- , und

A die Gruppen

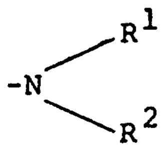 , 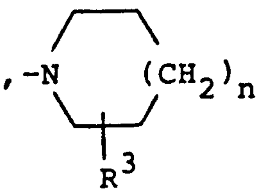 oder 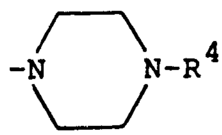

bedeuten, worin

R ein Wasserstoffatom oder eine Alkylgruppe mit 1 bis 3 Kohlenstoffatomen,

$R^1$ und $R^2$, die gleich oder voneinander verschieden sein können, ein Wasserstoffatom, einen Alkylrest mit 1 bis 3 Kohlenstoffatomen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,

n die Zahlen 0, 1 oder 2,

$R^3$ ein Wasserstoffatom, eine Hydroxygruppe, eine Alkylgruppe mit 1 bis 3 Kohlenstoffatomen oder eine Gruppe der Formel

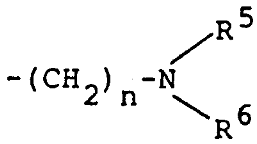 ,

worin n wie oben definiert ist, und $R^5$ und $R^6$ einen Alkylrest mit 1 bis 3 Kohlenstoffatomen bedeuten und

$R^4$ eine gerade oder verzweigte Alkylgruppe mit 1 bis 3 Kohlenstoffatomen oder eine Phenylalkylgruppe mit insgesamt 7 bis 9 Kohlenstoffatomen darstellen, und ihrer pharmakologisch verträglicher Salze mit anorganischen oder organischen Säuren, dadurch gekennzeichnet, daß

a.) eine Verbindung der allgemeinen Formel II

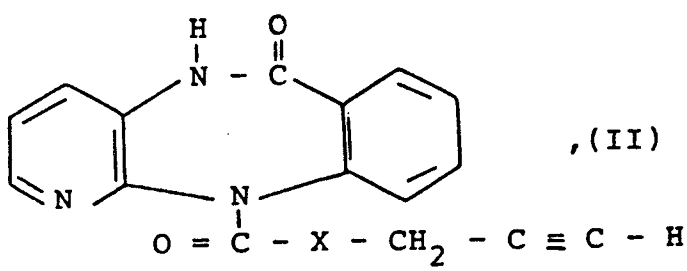 ,(II)

in der X wie oben angegeben definiert ist, mit einem Amin der Formeln

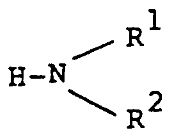,
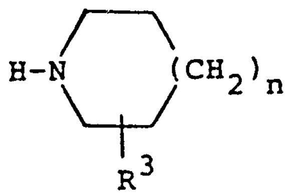,
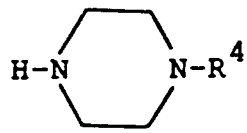

(IIIa) (IIIb) (IIIc)

worin $R^1$, $R^2$, $R^3$ und $R^4$ wie oben definiert sind, oder mit einem Salz dieses Amins in Gegenwart von Formaldehyd oder Paraformaldehyd und, gegebenenfalls, in Gegenwart katalytischer Mengen von Salzen, in organischen Lösungsmitteln bei Temperaturen bis zum Siedepunkt des Reaktionsgemisches umgesetzt oder

b.) zur Herstellung von Verbindungen der allgemeinen Formel I, in der X die Gruppe

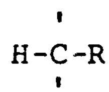

mit den für R oben angegebenen Bedeutung darstellt, das Dilithiumsalz der Verbindung der Formel IV

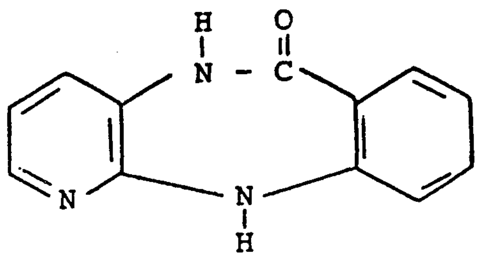

,(IV)

mit einem Ester der allgemeinen Formel V

$A\text{-}CH_2\text{-}C{\equiv}C\text{-}CH_2\text{-}CHR\text{-}COOR^7$ ,(V)

in der A und R wie oben definiert sind und $R^7$ einen Alkylrest mit 1 bis 10 Kohlenstoffatomen, einen Phenylalkylrest mit insgesamt 7 bis 10 Kohlenstoffatomen oder die Phenylgruppe bedeutet, in einem organischen Lösungsmittel bei Temperaturen zwischen -60 °C und +20 °C umgesetzt, und, gewünschtenfalls, anschließend eine so erhaltene Verbindung der allgemeinen Formel I in ihre Isomere aufgetrennt und/oder in ihre Salze mit anorganischen oder organischen Säuren übergeführt wird.

**Patentansprüche für folgenden Vertragstaat: AT**

1. Verfahren zur Herstellung von in 11-Stellung substituierten 5,11-Dihydro-6H-pyrido[2,3-b][1,4]-benzodiazepin-6-onen der allgemeinen Formel I

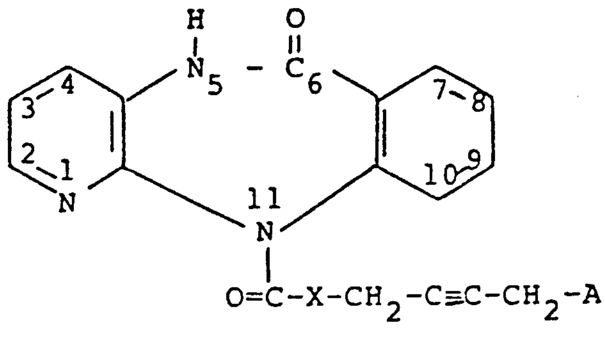

,(I)

in der

X die Gruppen

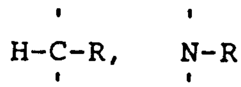

oder -0- , und

A die Gruppen

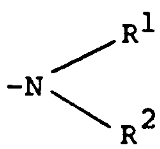

,

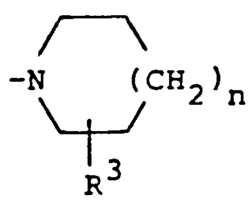

oder

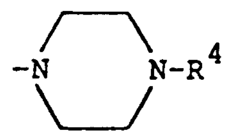

bedeuten, worin

R ein Wasserstoffatom oder eine Alkylgruppe mit 1 bis 3 Kohlenstoffatomen,

$R^1$ und $R^2$, die gleich oder voneinander verschieden sein können, ein Wasserstoffatom, einen Alkylrest mit 1 bis 3 Kohlenstoffatomen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,

n die Zahlen 0, 1 oder 2,

$R^3$ ein Wasserstoffatom, eine Hydroxygruppe, eine Alkylgruppe mit 1 bis 3 Kohlenstoffatomen oder eine Gruppe der Formel

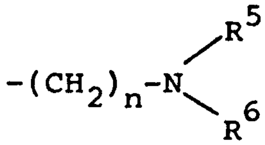

,

worin n wie oben definiert ist, und $R^5$ und $R^6$ einen Alkylrest mit 1 bis 3 Kohlenstoffatomen bedeuten und

$R^4$ eine gerade oder Verzweigte Alkylgruppe mit 1 bis 3 Kohlenstoffatomen oder eine Phenylalkylgruppe mit insgesamt 7 bis 9 Kohlenstoffatomen darstellen, und ihrer pharmakologisch verträglicher Salze

mit anorganischen oder organischen Säuren, dadurch gekennzeichnet, daß

a.) eine Verbindung der allgemeinen Formel II

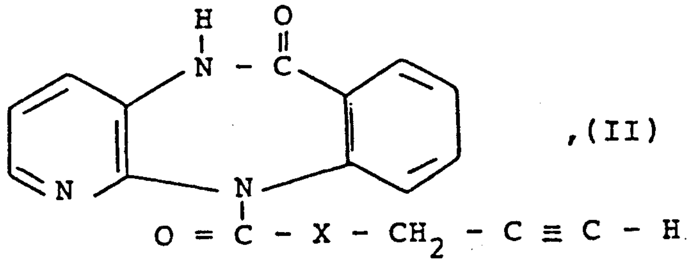
,(II)

in der X wie oben angegeben definiert ist, mit einem Amin der Formeln

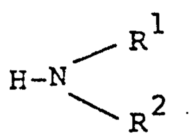
,
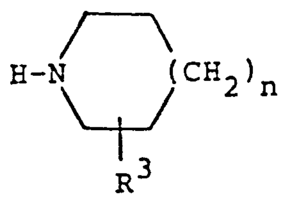
,
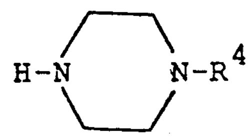

(IIIa) (IIIb) (IIIc)

worin $R^1$ $R^2$, $R^3$ und $R^4$ wie oben definiert sind, oder mit einem Salz dieses Amins in Gegenwart von Formaldehyd oder Paraformaldehyd und, gegebenenfalls, in Gegenwart katalytischer Mengen von Salzen, in organischen Lösungsmitteln bei Temperaturen bis zum Siedepunkt des Reaktionsgemisches umgesetzt oder

b.) zur Herstellung von Verbindungen der allgemeinen Formel I, in der X die Gruppe

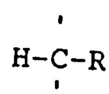

mit den für R oben angegebenen Bedeutung darstellt, das Dilithiumsalz der Verbindung der Formel IV

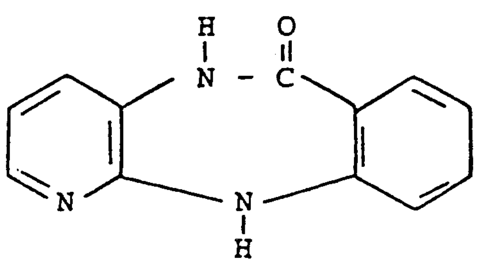
,(IV)

mit einem Ester der allgemeinen Formel V

$A\text{-}CH_2\text{-}C{\equiv}C\text{-}CH_2\text{-}CHR\text{-}COOR^7$ ,(V)

in der A und R wie oben definiert sind und $R^7$ einen Alkylrest mit 1 bis 10 Kohlenstoffatomen, einen Phenylalkylrest mit insgesamt 7 bis 10 Kohlenstoffatomen oder die Phenylgruppe bedeutet, in einem organischen Lösungsmittel bei Temperaturen zwischen -60 °C und +20 °C umgesetzt, und, gewünschtenfalls, anschließend eine so erhaltene Verbindung der allgemeinen Formel I in ihre Isomere

aufgetrennt und/oder in ihre Salze mit anorganischen oder organischen Säuren übergeführt wird.

**2.** Verfahren gemäß Anspruch 9a, dadurch gekennzeichnet, daß die Umsetzung von Verbindungen der allgemeinen Formel II mit den Aminen oder ihren Salzen der allgemeinen Formeln IIIa, IIIb oder IIIc in cyclischen Ethern oder in Alkoholen in Gegenwart von Kupfer(I)chlorid oder Eisen(II)chlorid erfolgt.

**3.** Verfahren gemäß Anspruch 9b, dadurch gekennzeichnet, daß als Lösungsmittel Tetrahydrofuran, Ether oder Hexan eingesetzt werden und das Dilithiumsalz kurz vor der Umsetzung in denselben Lösungsmitteln durch die Einwirkung von n-Butyllithium, gegebenenfalls in Gegenwart von Tetramethylendiamin, tertiär-Butyllithium, Lithiumdiisopropylamid, Lithiumdicyclohexylamid oder Lithiumphenyl auf die Verbindung der Formel IV bei Temperaturen zwischen -60 und 0 ° C erzeugt wird.

**Claims**

**Claims for the following Contracting States: BE, CH, DE, FR, GB, IT, LI, LU, NL, SE**

**1.** 5,11-Dihydro-6H-pyrido[2,3-b][1,4]benzodiazepin-6-ones substituted in the 11- position of general formula I

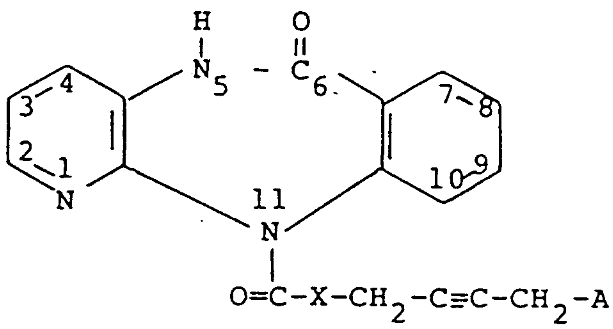

(I)

wherein

X represents the groups

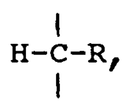,
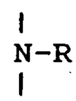

or -O-, and

A represents the groups

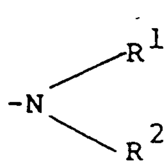,
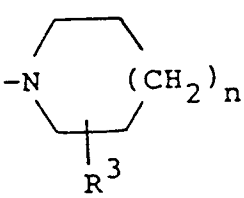
or
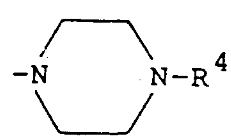

wherein

R represents a hydrogen atom or an alkyl group with 1 to 3 carbon atoms,

$R^1$ and $R^2$, which may be identical or different, represent a hydrogen atom, an alkyl group with 1 to 3 carbon atoms, a phenylalkyl group with a total of 7 to 9 carbon atoms, a 5 to 7 membered cycloalkyl group optionally substituted by a hydroxy group,

n represents the number 0, 1 or 2,

$R^3$ represents a hydrogen atom, a hydroxy group, an alkyl group with 1 to 3 carbon atoms or a group of formula

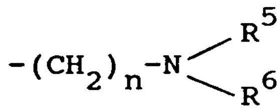

wherein n is defined as hereinbefore, and $R^5$ and $R^6$ represent an alkyl group with 1 to 3 carbon atoms, and

$R^4$ represents a straight-chained or branched alkyl group with 1 to 3 carbon atoms or a phenylalkyl group with a total of 7 to 9 carbon atoms,

the isomers and the pharmacologically acceptable salts thereof with inorganic or organic acids.

**2.** 5,11-Dihydro-11-[1-oxo-2-methyl-6-(4-methyl-1-piperazinyl)-4-hexynyl]-6H-pyrido[2,3-b][1,4]-benzodiazepin-6-one and the pharmacologically acceptable acid addition salts thereof with inorganic or organic acids.

**3.** 5,11-Dihydro-11-[1-oxo-6-(1-piperidinyl)-4-hexynyl]-6H-pyrido[2,3-b][1,4]benzodiazepin-6-one and the pharmacologically acceptable acid addition salts thereof with inorganic or organic acids.

**4.** 5,11-Dihydro-11-[1-oxo-6-(hexahydro-1H-azepin-1-yl)-4-hexynyl]-6H-pyrido[2,3-b][1,4]benzodiazepin-6-one and the pharmacologically acceptable acid addition salts thereof with inorganic or organic acids.

**5.** Pharmaceutical compositions containing a compound of general formula I as claimed in claim 1 together with conventional carriers and/or excipients.

**6.** Pharmaceutical compositions containing 5,11-dihydro-11-[1-oxo-2-methyl-6-(4-methyl-1-piperazinyl)-4-hexynyl]-6H-pyrido[2,3-b][1,4]benzodiazepin-6-one and/or the pharmacologically acceptable acid addition salts thereof with inorganic or organic acids together with conventional excipients and additives.

**7.** Use of 5,11-dihydro-11-[1-oxo-2-methyl-6-(4-methyl-1-piperazinyl)-4-hexynyl]-6H-pyrido[2,3-b][1,4]-benzodiazepin-6-one and/or the pharmacologically acceptable acid addition salts thereof with inorganic or organic acids for preparing a pharmaceutical composition with antithrombotic properties for the treatment of thromboembolic diseases.

**8.** Use of compounds of claims 1, 3 and 4 for preparing a pharmaceutical composition for the treatment of bradycardia and bradyarrhythmia.

**9.** Process for preparing 5,11-dihydro-6H-pyrido[2,3-b][1,4]benzodiazepin-6-ones substituted in the 11-position of general formula I

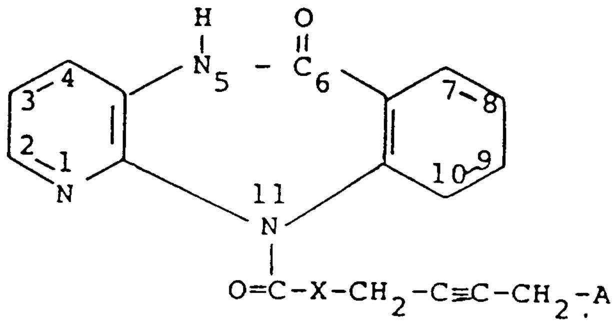

(I)

wherein

X represents the groups

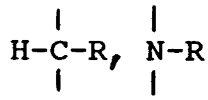

or -O-, and

A represents the groups

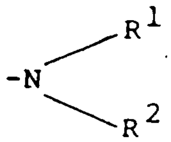, 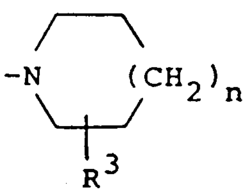 or 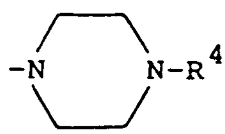

wherein

R represents a hydrogen atom or an alkyl group with 1 to 3 carbon atoms,

$R^1$ and $R^2$, which may be identical or different, represent a hydrogen atom, an alkyl group with 1 to 3 carbon atoms, a phenylalkyl group with a total of 7 to 9 carbon atoms, a 5 to 7 membered cycloalkyl group optionally substituted by a hydroxy group,

n represents the number 0, 1 or 2,

$R^3$ represents a hydrogen atom, a hydroxy group, an alkyl group with 1 to 3 carbon atoms or a group of formula

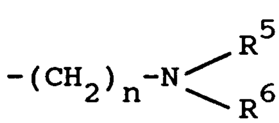

wherein n is defined as hereinbefore, and $R^5$ and $R^6$ represent an alkyl group with 1 to 3 carbon atoms, and

$R^4$ represents a straight-chained or branched alkyl group with 1 to 3 carbon atoms or a phenylalkyl group with a total of 7 to 9 carbon atoms, the isomers and the pharmacologically acceptable salts thereof with inorganic or organic acids, characterised in that

a) a compound of general formula II

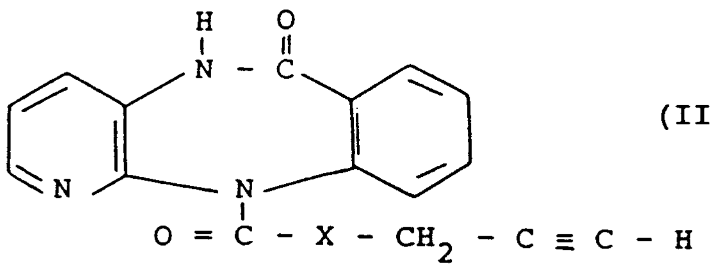

(II)

wherein X is defined as hereinbefore, is reacted with an amine of formula

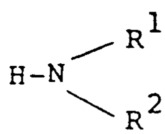

,

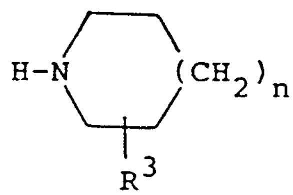

,

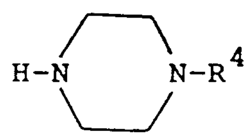

(IIIa) (IIIb) (IIIc)

wherein $R^1$, $R^2$, $R^3$ and $R^4$ are as hereinbefore defined, or with a salt of this amine in the presence of formaldehyde or paraformaldehyde and optionally in the presence of catalytic quantities of salts, in organic solvents at temperatures up to the boiling point of the reaction mixture, or

b) in order to prepare compounds of general formula I wherein X represents the group

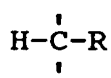

wherein R is as hereinbefore defined, the dilithium salt of the compound of formula IV

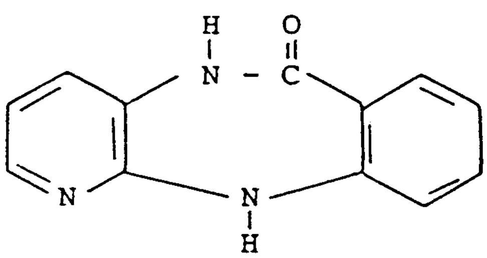

(IV)

is reacted with an ester of general formula V

$A-CH_2-C\equiv C-CH_2-CHR-COOR^7$ (V)

wherein A and R are as hereinbefore defined and $R^7$ represents an alkyl group with 1 to 10 carbon atoms, a phenylalkyl group with a total of 7 to 10 carbon atoms or a phenyl group, in an organic solvent at temperatures of between -60 °C and +20 °C

and, if desired, a compound of general formula I thus obtained is subsequently separated into the isomers thereof and/or converted into the salts thereof with inorganic or organic acids.

**Claims for the following Contracting State: AT**

**1.** Process for preparing 5,11-dihydro-6H-pyrido[2,3-b][1,4]benzodiazepin-6-ones substituted in the 11-position of general formula I

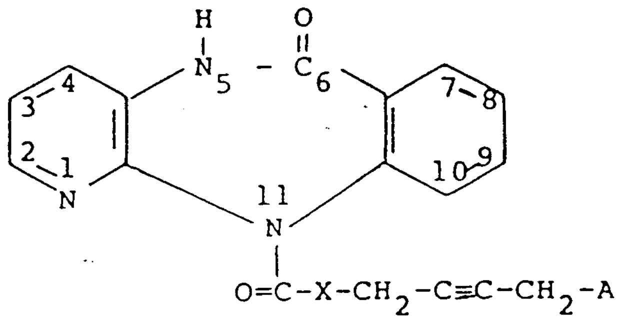

(I)

wherein

X represents the groups

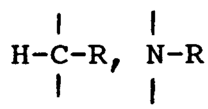

or -O-, and

A represents the groups

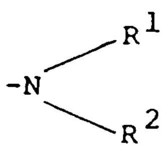 , 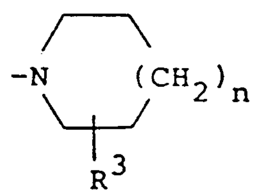 or 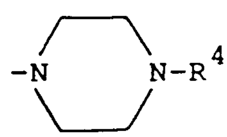

wherein

R represents a hydrogen atom or an alkyl group with 1 to 3 carbon atoms,

$R^1$ and $R^2$, which may be identical or different, represent a hydrogen atom, an alkyl group with 1 to 3 carbon atoms, a phenylalkyl group with a total of 7 to 9 carbon atoms, a 5 to 7 membered cycloalkyl group optionally substituted by a hydroxy group,

n represents the number 0, 1 or 2,

$R^3$ represents a hydrogen atom, a hydroxy group, an alkyl group with 1 to 3 carbon atoms or a group of formula

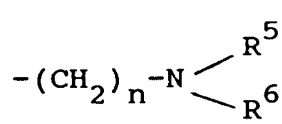

wherein n is defined as hereinbefore, and $R^5$ and $R^6$ represent an alkyl group with 1 to 3 carbon atoms, and

$R^4$ represents a straight-chained or branched alkyl group with 1 to 3 carbon atoms or a phenylalkyl group with a total of 7 to 9 carbon atoms, and the pharmacologically acceptable salts thereof with inorganic or organic acids, characterised in that

a) a compound of general formula II

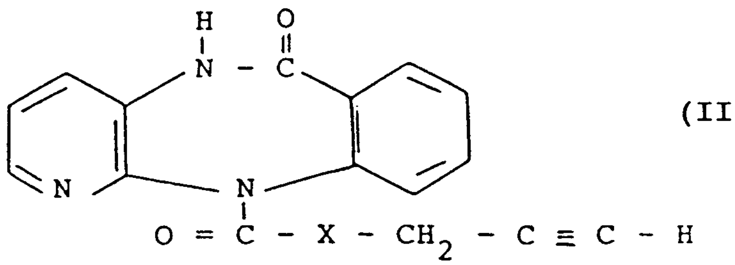

(II)

wherein X is defined as hereinbefore, is reacted with an amine of formula

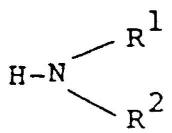 , 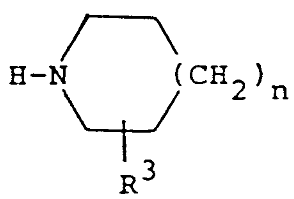 , 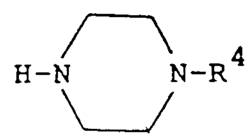

(IIIa) (IIIb) (IIIc)

wherein $R^1$, $R^2$, $R^3$ and $R^4$ are as hereinbefore defined, or with a salt of this amine in the presence of formaldehyde or paraformaldehyde and optionally in the presence of catalytic quantities of salts, in organic solvents at temperatures up to the boiling point of the reaction mixture, or

b) in order to prepare compounds of general formula I wherein X represents the group

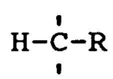

wherein R is as hereinbefore defined, the dilithium salt of the compound of formula IV

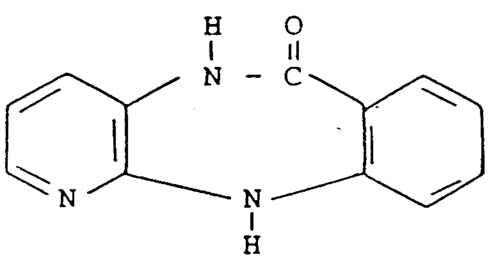

(IV)

is reacted with an ester of general formula V

$A-CH_2-C\equiv C-CH_2-CHR-COOR^7$ (V)

wherein A and R are as hereinbefore defined and $R^7$ represents an alkyl group with 1 to 10 carbon atoms, a phenylalkyl group with a total of 7 to 10 carbon atoms or a phenyl group, in an organic solvent at temperatures of between -60 °C and +20 °C

and, if desired, a compound of general formula I thus obtained is subsequently separated into the isomers thereof and/or converted into the salts thereof with inorganic or organic acids.

2. Process as claimed in claim 9a[sic], characterised in that the reaction of compounds of general formula II with the amines or salts thereof of general formulae IIIa, IIIb or IIIc is effected in cyclic ethers or in alcohols in the presence of copper(I) chloride or iron(II) chloride.

3. Process as claimed in claim 9b[sic], characterised in that tetrahydrofuran, ether or hexane is used as solvent and the dilithium salt is prepared shortly before the reaction in the same solvents by the action of n-butyl-lithium, optionally in the presence of tetramethylenediamine, tert.butyl-lithium, lithium diisopropylamide, lithium dicyclohexylamide or lithium phenyl, on the compound of formula IV at temperatures of between -60 and 0° C.

**Revendications**
**Revendications pour les Etats contractants suivants: BE, CH, DE, FR, GB, IT, LI, LU, NL, SE**

1. 5,11-dihydro-6H-pyrido[2,3-b][1,4]benzodiazépine-6-ones substituées en position 11 de formule générale I

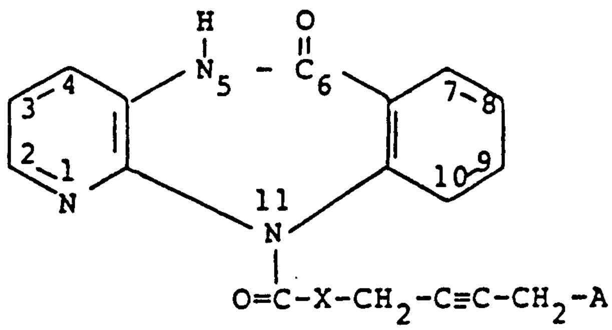

(I)

dans laquelle
X représente les groupes

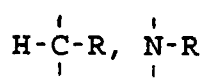

ou -O-, et
A représente les groupes

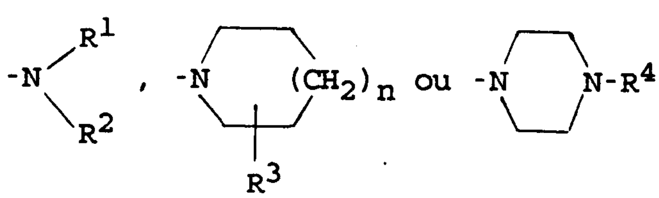

dans lesquels
R représente un atome d'hydrogène ou un groupe alkyle contenant 1 à 3 atomes de carbone,
$R^1$ et $R^2$, qui peuvent être identiques ou différents, représentent un atome d'hydrogène, un reste alkyle contenant 1 à 3 atomes de carbone, un reste phénylalkyle contenant au total 7 à 9 atomes de carbone, un reste cycloalkyle de 5 à 7 chaînons éventuellement substitué par un groupe hydroxyle, n représente les nombres 0, 1 ou 2,
$R^3$ représente un atome d'hydrogène, un groupe hydroxyle, un groupe alkyle de 1 à 3 atomes de carbone ou un groupe de formule

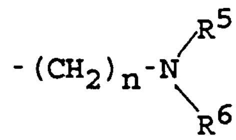 ,

dans laquelle n est défini comme ci-dessus, et $R^5$ et $R^6$ représentent un reste alkyle de 1 à 3 atomes de carbone et
$R^4$ représente un groupe alkyle linéaire ou ramifié de 1 à 3 atomes de carbone ou un groupe phénylalkyle contenant au total 7 à 9 atomes de carbone, leurs isomères et leurs sels, compatibles du point de vue pharmacologique, avec des acides minéraux ou organiques.

2. 5,11-dihydro-11-[1-oxo-2-méthyl-6-(4-méthyl-1-pipérazinyl)-4-hexinyl]-6H-pyrido[2,3-b][1,4]-benzodiazépine-6-one et ses sels d'addition d'acide, compatibles du point de vue pharmacologique, avec des acides minéraux ou organiques.

3. 5,11-dihydro-11-[1-oxo-6-(1-pipéridinyl)-4-hexinyl] -6H-pyrido[2,3-b][1,4]benzodiazépine-6-one et ses sels, compatibles du point de vue pharmacologique, avec des acides minéraux ou organiques.

4. 5,11-dihydro-11-[1-oxo-6-(hexahydro-1H-azépine-1-yl)-4-hexinyl]-6H-pyrido[2,3-b][1,4]benzodiazépine-6-one et ses sels, compatibles du point de vue pharmacologique, avec des acides minéraux ou organiques.

5. Médicament contenant un composé de formule générale I selon la revendication 1 ainsi que des véhicules et/ou adjuvants habituels.

6. Médicament contenant de la 5,11-dihydro-11-[1-oxo-2-méthyl-6-(4-méthyl-1-pipérazinyl)-4-hexinyl]-6H-pyrido [2,3-b][1,4]benzodiazépine-6-one et/ou ses sels d'addition d'acide, compatibles du point de vue pharmacologique, avec des acides minéraux ou organiques, ainsi que des adjuvants ou additifs habituels.

7. Utilisation de la 5,11-dihydro-11-[1-oxo-2-méthyl-6-(4-méthyl-1-pipérazinyl)-4-hexinyl]-6H-pyrido[2,3-b]-[1,4] benzodiazépine-6-one et/ou de ses sels d'addition d'acide, compatibles du point de vue pharmacologique, avec des acides minéraux ou organiques pour la préparation d'un médicament à propriétés antithrombotiques pour le traitement de maladies thromboemboliques.

8. Utilisation de composés des revendications 1, 3 et 4 pour la préparation d'un médicament pour le traitement des bradycardies et des bradyarhythmies.

9. Procédé de préparation de 5,11-dihydro-6H-pyrido[2,3-b][1,4]benzodiazépine-6-ones substituées en position 11 de formule générale I

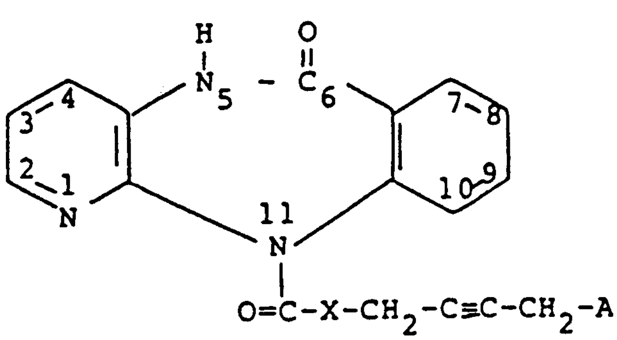

(I)

dans laquelle
X représente les groupes

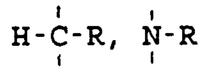

ou -O-, et
A représente les groupes

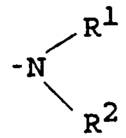 , 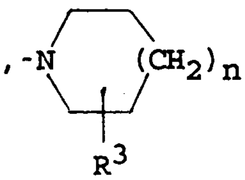 ou 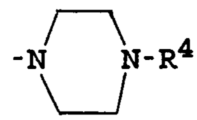

dans lesquels
R représente un atome d'hydrogène ou un groupe alkyle contenant 1 à 3 atomes de carbone,
$R^1$ et $R^2$, qui peuvent être identiques ou différents, représentent un atome d'hydrogène, un reste alkyle contenant 1 à 3 atomes de carbone, un reste phénylalkyle contenant au total 7 à 9 atomes de carbone, un reste cycloalkyle de 5 à 7 chaînons éventuellement substitué par un groupe hydroxyle, n représente les nombres 0, 1 ou 2,
$R^3$ représente un atome d'hydrogène, un groupe hydroxyle, un groupe alkyle de 1 à 3 atomes de carbone ou un groupe de formule

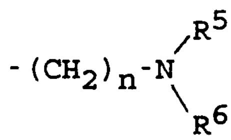 ,

dans laquelle n est défini comme ci-dessus, et $R^5$ et $R^6$ représentent un reste alkyle de 1 à 3 atomes de carbone et
$R^4$ représente un groupe alkyle linéaire ou ramifié de 1 à 3 atomes de carbone ou un groupe phénylalkyle contenant au total 7 à 9 atomes de carbone, et de leurs sels, compatibles du point de vue pharmacologique, avec des acides minéraux ou organiques, caractérisé en ce que

a) un composé de formule générale II

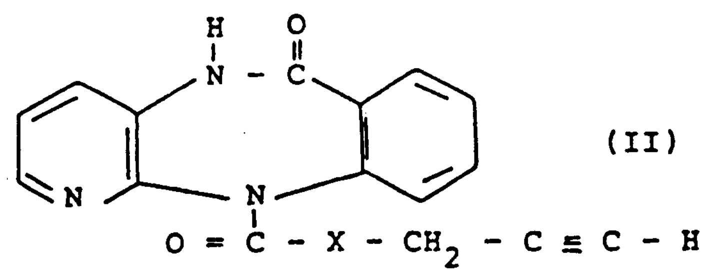

(II)

dans laquelle X est défini comme indiqué ci-dessus, est mis à réagir avec une amine de formule

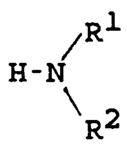 , 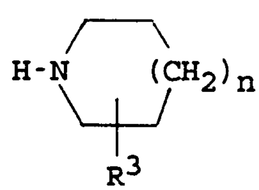 ou 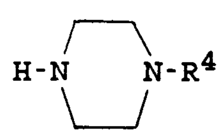

(IIIa) (IIIb) (IIIc)

dans laquelle $R^1$, $R^2$, $R^3$ et $R^4$ sont définis comme ci-dessus, ou avec un sel de cette amine en présence de formaldéhyde ou de paraformaldéhyde et, éventuellement, en présence de quantités catalytiques de sels, dans des solvants organiques à des températures atteignant le point d'ébullition du mélange réactionnel ou

b) pour la préparation de composés de formule générale I dans laquelle X représente le groupe

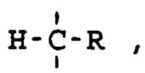

R ayant la signification donnée ci-dessus, le sel dilithien du composé de formule IV

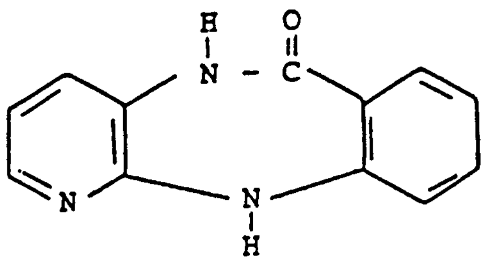

(IV)

est mis à réagir avec un ester de formule générale V

$A\text{-}CH_2\text{-}C\equiv C\text{-}CH_2\text{-}CHR\text{-}COOR^7$ (V)

dans laquelle A et R sont définis comme ci-dessus et $R^7$ représente un reste alkyle contenant 1 à 10 atomes de carbone, un reste phénylalkyle contenant au total 7 à 10 atomes de carbone ou le groupe phényle, dans un solvant organique à des températures comprises entre -60 °C et +20 °C, et, si on le souhaite, le composé de formule générale I ainsi obtenu est séparé en ses isomères et/ou est converti en ses sels avec des acides minéraux ou organiques.

**Revendications pour l'Etat contractant suivant: AT**

1. Procédé de préparation de 5,11-dihydro-6H-pyrido[2,3-b][1,4]benzodiazépine-6-ones substituées en position 11 de formule générale I

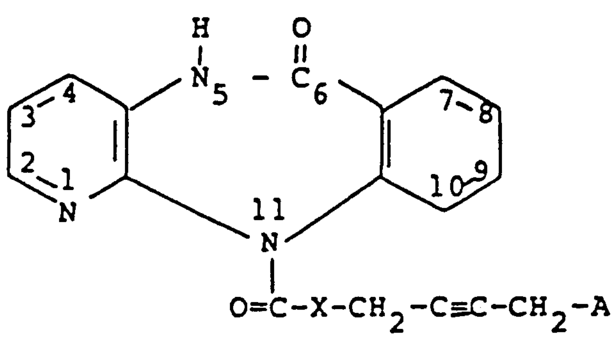

(I)

dans laquelle
X représente les groupes

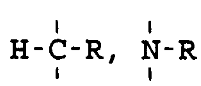

ou -O-, et
A représente les groupes

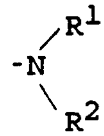 , 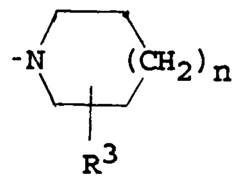 ou 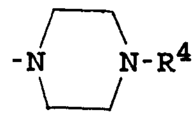

dans lesquels
R représente un atome d'hydrogène ou un groupe alkyle contenant 1 à 3 atomes de carbone,
$R^1$ et $R^2$, qui peuvent être identiques ou différents, représentent un atome d'hydrogène, un reste alkyle contenant 1 à 3 atomes de carbone, un reste phénylalkyle contenant au total 7 à 9 atomes de carbone, un reste cycloalkyle de 5 à 7 chaînons éventuellement substitué par un groupe hydroxyle,
n représente les nombres 0, 1 ou 2,
$R^3$ représente un atome d'hydrogène, un groupe hydroxyle, un groupe alkyle de 1 à 3 atomes de carbone ou un groupe de formule

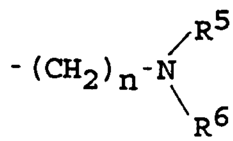 ,

dans laquelle n est défini comme ci-dessus, et $R^5$ et $R^6$ représentent un reste alkyle de 1 à 3 atomes de carbone et
$R^4$ représente un groupe alkyle linéaire ou ramifié de 1 à 3 atomes de carbone ou un groupe phénylalkyle contenant au total 7 à 9 atomes de carbone, et de leurs sels, compatibles du point de vue pharmacologique, avec des acides minéraux ou organiques, caractérisé en ce que

a) un composé de formule générale II

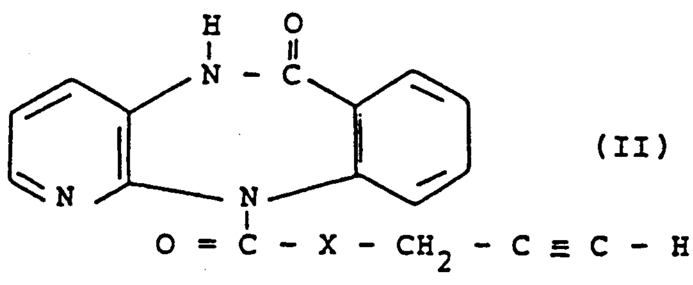

(II)

dans laquelle X est défini comme indiqué ci-dessus, est mis à réagir avec une amine de formule

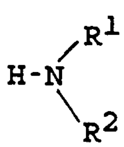 , 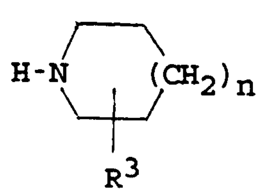 , 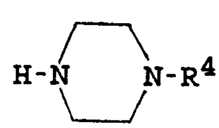

(IIIa) (IIIb) (IIIc)

dans laquelle $R^1$, $R^2$, $R^3$ et $R^4$ sont définis comme ci-dessus, ou avec un sel de cette amine en présence de formaldéhyde ou de paraformaldéhyde et, éventuellement, en présence de quantités catalytiques de sels, dans des solvants organiques à des températures atteignant le point d'ébullition du mélange réactionnel ou

b) pour la préparation de composés de formule générale I dans laquelle X représente le groupe

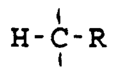 ,

R ayant la signification donnée ci-dessus, le sel dilithien du composé de formule IV

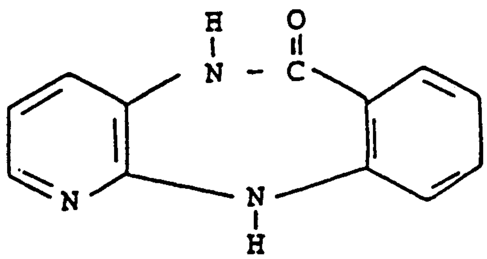

(IV)

est mis à réagir avec un ester de formule générale V

$A\text{-}CH_2\text{-}C{\equiv}C\text{-}CH_2\text{-}CHR\text{-}COOR^7$ (V)

dans laquelle A et R sont définis comme ci-dessus et $R^7$ représente un reste alkyle contenant 1 à 10 atomes de carbone, un reste phénylalkyle contenant au total 7 à 10 atomes de carbone ou le groupe phényle, dans un solvant organique à des températures comprises entre -60 ° C et + 20 ° C, et, si on le souhaite, le composé de formule générale I ainsi obtenu est séparé en ses isomères et/ou est converti en ses sels avec des acides minéraux ou organiques.

**2.** Procédé selon la revendication 1a, caractérisé en ce que la réaction de composés de formule générale II avec les amines ou leurs sels de formule générale IIIa, IIIb ou IIIc a lieu dans des éthers cycliques ou dans des alcools en présence de chlorure de cuivre (I) ou de chlorure de fer (II).

**3.** Procédé selon la revendication 1b, caractérisé en ce que le tétrahydrofuranne, un éther ou l'hexane est utilisé comme solvant et le sel dilithien est préparé peu avant la réaction dans les mêmes solvants par l'action du n-butyllithium, éventuellement en présence de tétraméthylènediamine, de tert.butyllithium, de diisopropylamidure de lithium, de dicyclohexylamidure de lithium ou de phényllithium sur le composé de formule IV à des températures comprises entre -60 ° C et 0 ° C.